Europäisches Patentamt

European Patent Office

Office européen des brevets

(19)

(11) Numéro de publication: **0 095 952**
A2

(12)

# DEMANDE DE BREVET EUROPEEN

(21) Numéro de dépôt: **83400899.7**

(22) Date de dépôt: **04.05.83**

(51) Int. Cl.³: **C 07 D 209/28,** C 07 C 91/10

(30) Priorité: **04.05.82 FR 8207757**

(71) Demandeur: **Bouchara, Emile, Dr., 75 bis, Avenue Foch, F-75116 Paris (FR)**

(43) Date de publication de la demande: **07.12.83 Bulletin 83/49**

(72) Inventeur: **Bouchara, Emile, Dr., 75 bis, Avenue Foch, F-75116 Paris (FR)**

(74) Mandataire: **Burtin, Jean-François, 5 Bis, rue Parmentier, F-92200 Neuilly S/Seine (FR)**

(84) Etats contractants désignés: **BE CH DE FR GB IT LI NL**

(54) **Nouveau sel d'indométacine, son procédé de préparation et les compositions pharmaceutiques en renfermant.**

(57) L'invention se rapporte à un nouveau sel d'indométacine soluble dans l'eau et dont les solutions aqueuses sont stables.

L'invention se rapporte à l'indométacinate de N-méthyl-glucamine se présentant sous forme de cristaux pratiquement incolores de point de fusion 144-146°.

Ce nouveau sel trouve un emploi comme principe actif de compositions pharmaceutiques liquides, notamment pour l'usage par voie parentérale.

EP 0 095 952 A2

La présente invention se rapporte à un nouveau sel d'acide arylacétique soluble dans l'eau et stable en solution.

L'invention concerne plus particulièrement un nouveau sel d'acide [1-(p-chlorobenzoyl)[- méthoxy indolyl- 3]acétique ou indométacine avec une base organique.

Elle a spécifiquement pour objet un sel cristallisé d'indométacine et de N-méthyl glucamine présentant un point de fusion de 144-146°.

De nombreux auteurs se sont effectivement penchés sur la possibilité d'obtenir un sel soluble et stable dans l'eau d'indométacine. La plupart des essais ont été décevants car l'indométacine s'hydrolyse très rapidement en milieu légèrement basique pour donner de l'acide p.chlorobenzoique.

De ce fait toutes les tentatives faites avec des dérivés alcalins, alcalinoterreux fournissent des sels peu ou pas solubles dans l'eau, ou fournissant rapidement des précipités de produits d'hydrolyse, insolubles dans l'eau. C'est ainsi que H. KRASOWSKA (Acta Pharm. Jugosl. 24 (1974) 193) écrit que la rapidité de la décomposition de l'indométacine avec l'augmentation du pH exclut la préparation d'une solution stable contenant un sel d'indométacine et qu'en outre la décomposition de l'indométacine en solution est catalysée par les ions OH ainsi que par les agents tampons.

Par ailleurs, la purification de l'indométacine et de ses dérivés est difficile et entraîne des chutes de rendement considérables.

Une nouvelle approche de ce problème a été réalisée par les travaux de A. BELLIGNO et Coll. dans Clinica Terapeutica 59 (1971) 483. Ces auteurs ont mis au point un produit dénommé sel d'indométacine avec la N- méthylglucamine qui se présente sous forme d'une poudre jaune-citron, microcristalline, très hygroscopique. Les solutions aqueuses de ce produit sont sensiblement alcalines.

En outre, le produit décrit par A. BELLIGNO possède un point de fusion de 94-95° et une microanalyse complètement discordante. La préparation de ce produit s'effectuait en milieu parfaitement anhydre.

Il est donc douteux que le produit dénommé par ces auteurs sel d'indométacine avec la N-méthylglucamine corresponde bien à la composition chimique annoncée.

En effet, les travaux de la demanderesse ont montré que la salification de l'indométacine par une base assez forte comme la N-méthylglucamine nécessitait un mode opératoire particulier, pour éviter toute hydrolyse de l'indométacine. Ils ont montré également qu'il était néanmoins possible d'opérer en milieu aqueux et que le sel ainsi obtenu se séparait sous forme cristallisée. Les cristaux ainsi obtenus sont pratiquement incolores. Ils présentent un point de fusion considérablement plus élevé que celui décrit pour le produit BELLIGNO. Ils ne retiennent pas de solvant et l'analyse montre que le produit est parfaitement homogène en chromatographie en couche mince et répond très exactement aux exigences de la microanalyse. Le produit ainsi obtenu est parfaitement stable à l'état sec.

Enfin, l'indométacinate de N-méthylglucamine ainsi obtenu est très soluble dans l'eau, donne des solutions stables qui ne s'altèrent pas à la longue.

Il est donc possible de réaliser des solutions d'indométacine et notamment des solutions injectables qui permettent le traitement des formes aiguës du rhumatisme articulaire.

L'invention concerne aussi un procédé d'obtention de l'indométacine de N-méthylglucamine de point de fusion 144-146° qui consiste à dissoudre l'indométacine dans un solvant cétonique, à ajouter une solution aqueuse concentrée de N- méthylglucamine en quantité stoechiométrique puis d'ajouter une nouvelle quantité de solvant cétonique. Le sel de N- méthylglucamine précipite progressivement. On laisse reposer le milieu à température ordinaire, puis on sépare les cristaux que l'on essore et lave avec le solvant cétonique. Après séchage à poids constant, le rendement en indométacinate de N-méthylglucamine est voisin de la théorie.

D'une manière préférée, le solvant cétonique est un solvant miscible à l'eau comme l'acétone. On peut également utiliser la diethylcétone ou la méthyl éthylcétone.

L'invention se rapporte encore aux compositions pharmaceutiques renfermant l'indométacinate de N-méthylglucamine de point de fusion 144-146° en mélange ou en association avec un véhicule aqueux.

De préférence les compositions pharmaceutiques sont destinées à l'administration par voie parentérale comme la voie intraveineuse, sous-cutanée, intra-musculaire ou intra-articulaire. Elles peuvent également être utilisées pour la voie orale sous forme de solutions, de gouttes ou de sirops.

Les formes pharmaceutiques peuvent renfermer en outre des substances tampons, des agents diluants, des agents dispersants ou tensioactifs, des agents édulcorants ou de sapidité.

D'une manière préférée les solutions d'indométacinate de N-méthylglucamine sont ajustées à un pH compris entre 7,05 et 7,25 par addition d'un tampon phosphaté et à un point d'abaissement cryoscopique voisin de 0,5. Ainsi les injections des solutions d'indométacinate de N-méthylglucamine sont elles indolores et bien tolérées.

Il peut être avantageux d'ajouter aux solutions d'indométacinate de N-méthylglucamine un agent dispersant, comme par exemple la polyvinyl pyrolidone et notamment une polyvinyl pyrolidone de poids moléculaire compris entre 10.000 et 15.000 comme par exemple le produit vendu sous la marque POLYVIDONE K15 ou POLYVIDONE C15.

On peut également trouver intérêt à ajouter aux solutions d'indométacinate de N-méthylglucamine un agent diluant comme le mannitol ou l'inositol. En effet, les solutions d'indométacinate de N- méthylglucamine sont présentées de préférence sous forme de flacons lyophilisés et l'adjonction de tels diluants permet d'obtenir un culot de lyophilisation plus important, qui se détache plus facilement des parois du flacon et permet une mise en solution au moment de l'emploi,plus aisée.

La posologie unitaire est parallèle à celle de l'indométacine et les compositions pharmaceutiques renferment de préférence de 38 mg d'indométacinate de N-méthylglucamine de point de fusion 144-146° (ce qui correspond à 25 mg d'indométacine) à 116 mg d'indométacinate de N-méthylglucamine de point de fusion 144-146° (ce qui correspond à 75 mg d'indométacine).

Les exemples suivants illustrent l'invention sans toutefois la limiter.

Exemple I

Préparation de l'indométacinate de N-méthylglucamine.

On dissout 7g15 d'indométacine dans 250 ml d'acétone à 20°. On ajoute progressivement une solution de 1g95 de N-méthylglucamine dans 15 ml d'eau à 20°. Après addition et mélange complet on ajoute à nouveau 100 ml d'acétone. On refroidit le mélange à 0° puis on laisse reposer à température ordinaire pendant 24 heures. On sépare le précipité cristallin que l'on essore, lave à l'acétone et sèche sous vide jusqu'à poids constant.

On obtient ainsi 9 g d'indométacinate de N-méthylglucamine fondant à 144-146°.

Le produit est complètement désolvaté.

En chromatographie en couche mince il est homogène

Les spectres UV et IR sont concordants avec la structure

Analyse $C_{26} H_{33} O9N_2Cl = 552,99$

|  | C | H | N | Cl | O % |
|---|---|---|---|---|---|
| Calculé | 56,46 | 6,01 | 5,07 | 6,41 | 26,04 |
| Trouvé { | 56,25 | 6,05 | 4,94 | 6,68 | 25,85 |
| | 56,32 | 5,98 | 5,11 |  | 25,84 |

Exemple II

Préparation lyophilisée injectable.

Indométacinate de N- méthylglucamine 38g6

Polyvinyl pyrolidone commercialisée

sous la marque POVIDONE C15          10g

Tampon phosphaté    q.s.p.          1000ml

{ phosphate mono potassique          10g2 )

Solution de soude N'          52ml5 }

Eau    q.s.p.          1000ml }

pour 500  ampoules à lyophiliser la préparation est dissoute à nouveau dans 2 ml d'eau distillée pH spontané entre 7,1 et 7,2 point d'abaissement cryoscopique    Δ= 0,48

0,52

La solution ainsi obtenue est parfaitement limpide et ne fournit qu'une seule tache en chromatographie couche mince.

Exemple III

Préparation lyophilisée injectable.

| | |
|---|---|
| Indométacinate de N- méthylglucamine 38g6 | |
| Polyvinyl pyrolidone commercialisée | |
| sous la marque POVIDONE C15 | |
| Mannitol | .125g |
| Tampon phosphaté q.s.p. | 1000ml |

pour 500 ampoules lyophilisées de 2 ml.

le pH de la préparation après redissolution dans 2 ml d'eau distillée est de 7,12.

# REVENDICATIONS

1°) L'indométacinate de N-méthylglucamine se présentant sous forme de cristaux pratiquement incolores, solubles et stables dans l'eau et possédant un point de fusion F = 144-146°.

2°) Un procédé de préparation de l'indométacinate de N-méthylglucamine selon la revendication 1°) caractérisé en ce que l'on dissout l'indométacine dans un solvant cétonique miscible à l'eau, ajoute une solution aqueuse de N-méthylglucamine et précipite le sel formé par une nouvelle addition du solvant cétonique.

3°) Les compositions pharmaceutiques renfermant à titre de principe actif l'indométacinate de N-méthylglucamine selon la revendication 1° en mélange ou en association avec un véhicule aqueux.

4°) Les compositions pharmaceutiques selon la revendication 3°) renfermant en outre un agent diluant et/ou un agent dispersant et/ou un agent tampon.

5°) Les compositions pharmaceutiques selon l'une des revendications 3°) ou 4°) dans laquelle la teneur en composé selon la revendication 1°) varie de 38 mg à 116 mg par unité de prise.